# EUROPEAN PATENT APPLICATION

(11) **EP 4 831 774 A1**
(43) Date of publication of application: **28.10.2026**
(21) Application number: 25205540.5
(22) Date of filing: 30.09.2025
(51) Int. Cl.: G06F 3/01, A61B 5/00, A61B 6/46, G06F 3/0362

(54) **HAPTIC INTERFACE FOR A MEDICAL IMAGING DEVICE**

(71) Applicant: Siemens Healthineers AG, 91301 Forchheim (DE)
(72) Inventor: Achleitner, Roman, 91301 Forchheim (DE); Betz, Tobias, 91301 Forchheim (DE); Huther, Wolfgang, 90425 Nürnberg (DE)
(74) Representative: Siemens Healthineers Patent Attorneys

(57) **Abstract**

The invention relates to a haptic interface for a medical imaging device, the haptic interface comprising a base region and a contact region,
- wherein the haptic interface is configured for detecting a manual input action of a hand based on a tactile contact between the hand and the contact region, for generating a control signal for the medical imaging device based on the detected manual input action and for providing haptic feedback to the hand in response to the detected manual input action,
- wherein the contact region of the haptic interface comprises a top surface and a lateral surface, the top surface facing axially along a reference axis of the haptic interface,
- wherein the lateral surface is arranged circumferentially with respect to the reference axis between the base region and the top surface and facing radially away from the reference axis.

## Description

In one aspect the invention relates to a haptic interface for a medical imaging device. In other aspects, the invention relates to a medical imaging device and to a method for operating the haptic interface.

Staff shortages, a lack of experts, and an increasing ratio of elderly people are leading to high throughput and increasing workload in clinical environments. This is why "ease of use" and simplified interactions will be one of the key drivers in the future. Beyond this, the experts' use cases will remain relevant. Both need to be fostered by human-machine-interface (HMI) solutions.

Prior art related to this technical field is disclosed, for example, in US 11 925 486 B2.

The underlying technical problem of the invention is to provide an alternative and/or an improvement to already known solutions in the field of haptic interfaces for medical imaging devices. This problem is solved by the subject matter of the independent claims. The dependent claims are related to further aspects of the invention. Independent of the grammatical term usage, individuals with male, female or other gender identities are included within the term.

In one aspect the invention relates to a haptic interface for a medical imaging device, the haptic interface comprising a base region and a contact region,
- wherein the haptic interface is configured for detecting a manual input action of a hand based on a tactile contact between the hand and the contact region, for generating a control signal for the medical imaging device based on the detected manual input action and for providing haptic feedback to the hand in response to the detected manual input action,
- wherein the contact region of the haptic interface comprises a top surface and a lateral surface, the top surface facing axially along a reference axis of the haptic interface,
- wherein the lateral surface is arranged circumferentially with respect to the reference axis between the base region and the top surface and facing radially away from the reference axis.

In particular, the hand may be a human hand and/or a hand of a user of the haptic interface. The reference axis may extend in a direction facing the user. The circumferential arrangement of the lateral surface with respect to the reference axis may be, in particular, annular, cylindrical and/or prism-like. The contact region may have, for example, the shape of a rotary knob.

The haptic feedback may be active and/or adaptive. In particular, the haptic feedback may be provided by an electromechanical actuator, for example a vibration motor and/or a piezoelectric element. Adaptive haptic feedback may be a dynamic and/or context-sensitive form of tactile output that adjusts its characteristics, in particular such as intensity, frequency, duration, or pattern, in response to real-time user interactions, environmental conditions, and/or system configuration states.

In a further aspect, the haptic interface further comprises a visual display screen, the visual display screen being integrated into the top surface and facing axially along the reference axis. Through the visual display, the user may be informed about the functions and usage of the haptic interface, in particular in text form in the language set on the device. In a further aspect, the visual display screen is touch-sensitive.

In a further aspect, the haptic interface is further configured for receiving configuration data, for displaying a visual information indicative of the configuration data through the visual display screen and for generating the control signal for the medical imaging device based on the detected manual input action and the configuration data.

The configuration data may be generated based on a user interaction with a user interface, in particular a graphical user interface. The configuration data may be indicative of a function of the medical imaging device, the function being controlled by the haptic interface. The function may be related to a start and/or stop of an imaging scan, switching lasers on and/or off for patient positioning or interventional navigation, moving a tabletop and/or injecting a contrast agent. The user may flexibly select the desired functionality through the graphical user interface, for example on a separate touch display, before triggering it with the haptic interface. The visual information may comprise a decision support information indicative of a workflow action, in particular a recommended next workflow action, depending on the current status of the workflow, enabling even inexperienced staff to carry out scans quickly.

In a further aspect, when the manual input action is indicative of a rotary motion about the reference axis relative to the base region, the haptic feedback comprises a virtual haptic rotational response.

The control region may be configured to remain non-rotational about the reference axis relative to the base region during the manual input action. In particular, the tactile contact may occur between the hand and the lateral surface. The lateral surface may be configured to remain non-rotational about the reference axis relative to the base region during the manual input action. The top surface, in particular including the visual display screen, may be configured to remain non-rotational about the reference axis relative to the base region during the manual input action.

The visual information may be configured to remain non-rotational about the reference axis relative to the base region during the manual input action. This may be achieved, for example, through the top surface remaining non-rotational about the reference axis relative to the base region during the manual input action. Another option could be an adapting of the visual information depending on the rotary motion to keep the visual information non-rotational about the reference axis relative to the base region during the manual input action, when the visual display screen follows the rotary motion.

The manual input action may be indicative of a push motion, in particular along the reference axis, relative to the base region and/or a slide motion relative to the base region. The manual input action may be indicative of a joystick motion relative to the base region.

In a further aspect, the haptic feedback comprises a virtual haptic rotational resistance response to the rotary motion. The virtual haptic rotational resistance response may comprise, for example, a virtual snap point, a virtual end point, a virtual detent and/or a virtual spring loading. The virtual haptic rotational resistance response may be a virtual rotational constraint to the rotary motion.

In a further aspect, the haptic interface comprises a circumferential light-emitting indicator arranged coaxially with the reference axis, positioned at a greater radial distance from the reference axis than the lateral surface and facing axially along the reference axis,
- wherein the haptic interface is configured for providing visual feedback through the circumferential light-emitting indicator in response to the detected manual input action,
- wherein the lateral surface is arranged circumferential with respect to the reference axis between the circumferential light-emitting indicator and the top surface.

The circumferential light-emitting indicator may be static with respect to the base region and/or co-planar with the base. The light-emitting indicator may comprise, for example, an array of light emitting diodes, in particular arranged along a circumferential path. The circumferential path may be, for example, circular, half-circular or polygonal. The visual feedback may be active and/or adaptive. The visual feedback may be generated based on the configuration data. The haptic interface may be configured for providing a function-specific light pattern indicative of a selected function of the medical imaging device through the circumferential light-emitting indicator.

In another aspect, the invention relates to a medical imaging device, comprising the haptic interface according to one of the aspects of the invention and a gantry, wherein the base region of the haptic interface is integrated into a cover of the gantry.

In particular, a region of the cover adjacent to the base region may extend substantially planar in a plane that is substantially perpendicular to the reference axis. In particular, the haptic interface may comprise a faceplate, the faceplate extending substantially perpendicular to the reference axis and radially outward from the base region. A radially outer edge region of the faceplate may be configured for a connection to the cover. In a further aspect, the haptic interface may be located in a control room, the control room being spatially separated from a scan room, in which a gantry of the medical imaging device is located.

The medical imaging device may be, for example, a computed tomography device, a magnetic-resonance imaging device, an ultrasound imaging device, a PET imaging device, a SPECT imaging device, an X-ray imaging device, in particular a C-arm imaging device, a fluoroscopy imaging device or a cone beam CT device, and/or a combination thereof. The medical imaging device may further comprise a control device, in particular in form of a tablet computer. The control device may comprise a user interface, in particular the user interface for the user interaction for generating the configuration data.

In another aspect, the gantry comprises an opening into which an examination object is insertable along a system axis of the medical imaging device, wherein the reference axis of the haptic interface is parallel to the system axis.

In another aspect, the medical imaging device further comprises an examination table, the examination table comprising a tabletop, a table base and a table drive,
- wherein the tabletop of the examination table is insertable into the opening along the system axis through a translational movement of the tabletop relative to the table base along the system axis,
- wherein the table drive is configured to drive the translational movement based on the control signal,
- wherein the manual input action is indicative of a rotary motion about the reference axis relative to the base region,
- wherein the control signal is indicative of a velocity of the translational movement, the velocity being adjustable based on a deflection angle of the rotary motion.

In another aspect, the medical imaging device further comprises a control system, wherein the control system is configured for generating the configuration data for the haptic interface based on imaging workflow data. The control system may utilize imaging workflow data, for example, imaging scan protocol data, data regarding a position and/or configuration state of a contrast media injector, and/or a patient positioning data. The control system may receive sensor data, for example camera data, and/or user interaction data, for example from the user interface.

The control system may be configured for generating the configuration data and/or the decision support information based on the sensor data, the user interaction data and/or the imaging workflow data, in particular by applying a trained model to the sensor data, the user interaction data and/or the imaging workflow data. The trained model may be based on one or more of the following architectures: deep convolutional neural network, deep belief network, random forest, deep residual learning, deep reinforcement learning, recurrent neural network, Siamese network, generative adversarial network or auto-encoder. The control system may comprise, for example, a processor, in particular for generating and/or calculating data, and/or a data interface, in particular for receiving and/or providing data.

In another aspect, the invention relates to a method for operating the haptic interface according to one of the aspects of the invention, the method comprising:
- detecting the manual input action of the hand based on the tactile contact between the hand and the contact region of the haptic interface,
- generating the control signal for the medical imaging device based on the detected manual input action and
- providing the haptic feedback to the hand in response to the detected manual input action.

In another aspect, the method further comprises:
- receiving the configuration data,
- displaying the visual information indicative of the configuration data through the visual display screen,
- wherein the control signal for the medical imaging device is generated based on the detected manual input action and the configuration data.

For example, the control signal may initiate a positioning adjustment of the gantry and/or the examination table, such as moving the table longitudinally or tilting the gantry to achieve the desired imaging angle. In another example, the control signal may trigger a modification of imaging parameters, for example an adjustment of radiation dose, or setting of scan duration and/or resolution. The configuration data may define permissible ranges or presets for these parameters, which may then be applied in response to the manual input action.

Further, the control signal may be used to activate or deactivate auxiliary systems, such as contrast agent injection timing, patient communication systems, or environmental controls like lighting or acoustic shielding. In yet another example, the control signal may initiate a pre-scan calibration routine, including detector alignment, system warm-up, or phantom imaging for quality assurance. The configuration data may specify the type and sequence of calibration steps required for a particular imaging protocol.

In another aspect, the method further comprises:
- adjusting a velocity of a translational movement based on a deflection angle of the rotary motion,
- wherein the manual input action is indicative of the rotary motion about the reference axis relative to the base region,
- wherein the control signal is indicative of the velocity of the translational movement.

In particular, the velocity of the translational movement may increase with an increasing deflection angle of the rotary motion, for example within a predefined velocity range. The translational movement may be, for example, a translational movement of a tabletop of an examination table of the medical imaging device relative to a table base of the examination table of the medical imaging device, in particular along a system axis of the medical imaging device.

The translational movement may be horizontal. A direction of the translational movement, in particular whether the tabletop is moved into or out of the opening of the gantry, may be controlled based on a direction of the rotary motion. A running translational movement may be stopped, for example, by pushing the top surface of the control region along the reference axis toward the base region.

Based on the configuration data, an examination table control function of the haptic interface may be selectively switched between a horizontal table positioning function and a vertical table positioning function. If the vertical table positioning function is selected based on the configuration data, a direction of a vertical movement of the tabletop relative to the table base, in particular whether the tabletop is elevated or lowered, may be controlled based on a direction of the rotary motion.

In another aspect, when the deflection angle of the rotary motion transitions across a threshold angle, a virtual rotational snap point is provided, the threshold angle corresponding to a predefined velocity threshold of the translational movement. The haptic feedback may, for example, comprise virtual rotational snap points at transitions between different velocity levels and/or comprise a virtual end point for the rotary motion once a velocity limit is reached.

This enables a one-button solution that simplifies interactions while providing the capability to handle complex workflows. This contrasts with traditional multi-button controls and complex interfaces. The advantages include improved ease of use, enhanced user experience, seamless integration into the workflow and reduced training time. The proposed solution allows to reduce the large number of buttons of known human-machine-interfaces to a single button. The cleaning effort is massively reduced by minimizing the gaps and edges that are vulnerable to dirt.

In the context of the present invention, the expression "based on" can in particular be understood as meaning "using, inter alia". In particular, wording according to which a first feature is calculated (or generated, determined etc.) based on a second feature does not preclude the possibility of the first feature being calculated (or generated, determined etc.) based on a third feature. Some or all of the described solutions may be merely exemplary and the solutions may be varied by a person skilled in the art, without departing from the scope of the invention as it is specified by the claims.

Features related to the invention or to the technical background of the invention will be illustrated below with reference to the accompanying figures. The illustration in the figures is schematic and highly simplified and not necessarily to scale.
Fig. 1 shows a medical imaging device.
Fig. 2 shows the haptic interface.
Fig. 3 shows a flow chart for a method for operating a haptic interface.
Fig. 4 shows a flow chart for a method for operating a haptic interface for adjusting a velocity of a translational movement.

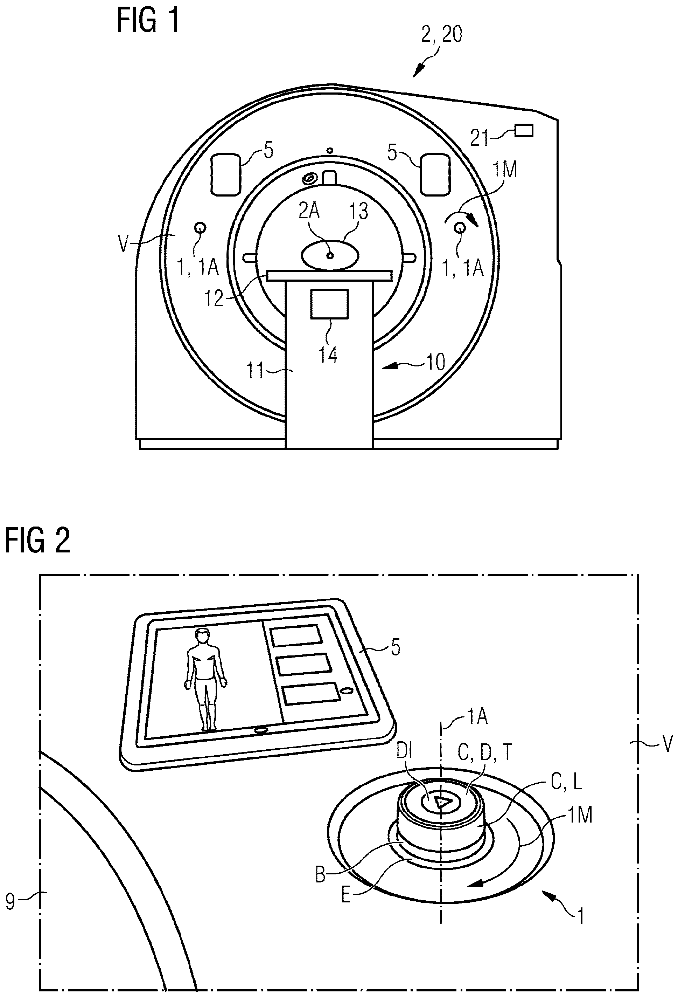

Fig. 1 shows the medical imaging device 2, comprising the haptic interface 1 and the gantry 20, wherein the base region B of the haptic interface 1 is integrated into a cover V of the gantry 20.

The gantry 20 comprises an opening 9 into which an examination object 13 is insertable along a system axis 2A of the medical imaging device 2, wherein the reference axis 1A of the haptic interface 1 is parallel to the system axis 2A.

The medical imaging device 2 further comprises an examination table 10, the examination table 10 comprising a tabletop 12, a table base 11 and a table drive 14,
- wherein the tabletop 12 of the examination table 10 is insertable into the opening 9 along the system axis 2A through a translational movement of the tabletop 12 relative to the table base 11 along the system axis 2A,
- wherein the table drive 14 is configured to drive the translational movement based on the control signal,
- wherein the manual input action is indicative of a rotary motion 1M about the reference axis 1A relative to the base region B,
- wherein the control signal is indicative of a velocity of the translational movement, the velocity being adjustable based on a deflection angle of the rotary motion 1M.

The medical imaging device 2 further comprises a control system 21, wherein the control system is configured for generating the configuration data for the haptic interface 1 based on imaging workflow data. The medical imaging device 2 further comprise the control device 5 in form of a tablet computer.

Fig. 2 shows the haptic interface 1 for the medical imaging device 2, the haptic interface 1 comprising a base region B and a contact region C,
- wherein the haptic interface 1 is configured for detecting S1 a manual input action of a hand based on a tactile contact between the hand and the contact region C, for generating S2 a control signal for the medical imaging device 2 based on the detected manual input action and for providing S3 haptic feedback to the hand in response to the detected manual input action,
- wherein the contact region C of the haptic interface 1 comprises a top surface T and a lateral surface L, the top surface T facing axially along a reference axis 1A of the haptic interface 1,
- wherein the lateral surface L is arranged circumferentially with respect to the reference axis 1A between the base region B and the top surface T and facing radially away from the reference axis 1A.

In the shown example, the haptic interface 1 further comprises a visual display screen D, the visual display screen D being integrated into the top surface T and facing axially along the reference axis 1A. In the shown example, the visual display screen D is touch-sensitive.

In the shown example, the haptic interface 1 is further configured for receiving S11 configuration data, for displaying a visual information DI indicative of the configuration data through the visual display screen D and for generating the control signal for the medical imaging device 2 based on the detected manual input action and the configuration data.

In the shown example, the manual input action is indicative of a rotary motion 1M about the reference axis 1A relative to the base region B and the haptic feedback comprises a virtual haptic rotational response. In the shown example, the haptic feedback comprises a virtual haptic rotational resistance response to the rotary motion 1M.

In the shown example, the haptic interface 1 comprises a circumferential light-emitting indicator E arranged coaxially with the reference axis 1A, positioned at a greater radial distance from the reference axis 1A than the lateral surface L and facing axially along the reference axis 1A,
- wherein the haptic interface 1 is configured for providing visual feedback through the circumferential light-emitting indicator E in response to the detected manual input action,
- wherein the lateral surface L is arranged circumferential with respect to the reference axis 1A between the circumferential light-emitting indicator E and the top surface T.

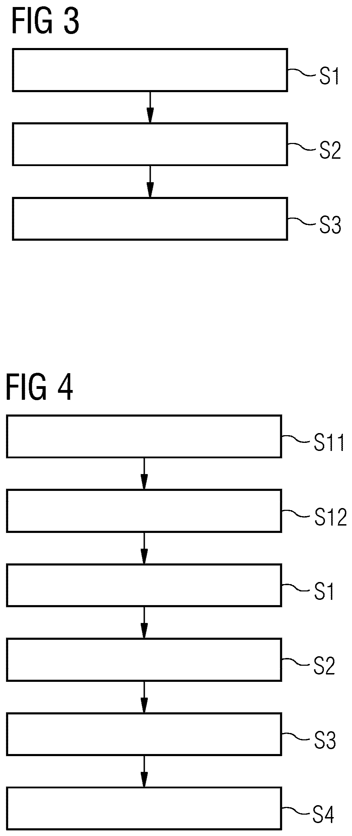

Fig. 3 shows a flow chart of a method for operating the haptic interface 1, the method comprising:
- detecting S1 the manual input action of the hand based on the tactile contact between the hand and the contact region C of the haptic interface 1,
- generating S2 the control signal for the medical imaging device 1 based on the detected manual input action and
- providing S3 the haptic feedback to the hand in response to the detected manual input action.

Fig. 4 shows a flow chart for a method for operating the haptic interface 1, the method comprising:
- receiving S11 the configuration data,
- displaying S12 the visual information DI indicative of the configuration data through the visual display screen D,
- wherein the control signal for the medical imaging device 2 is generated based on the detected manual input action and the configuration data.

The method further comprises:
- adjusting S4 a velocity of a translational movement based on a deflection angle of the rotary motion 1M,
- wherein the manual input action is indicative of the rotary motion 1M about the reference axis 1A relative to the base region B,
- wherein the control signal is indicative of the velocity of the translational movement.

## Claims

1. A haptic interface (1) for a medical imaging device (2), the haptic interface (1) comprising a base region (B) and a contact region (C),
- wherein the haptic interface (1) is configured for detecting (S1) a manual input action of a hand based on a tactile contact between the hand and the contact region (C), for generating (S2) a control signal for the medical imaging device (2) based on the detected manual input action and for providing (S3) haptic feedback to the hand in response to the detected manual input action,
- wherein the contact region (C) of the haptic interface (1) comprises a top surface (T) and a lateral surface (L), the top surface (T) facing axially along a reference axis (1A) of the haptic interface (1),
- wherein the lateral surface (L) is arranged circumferentially with respect to the reference axis (1A) between the base region (B) and the top surface (T) and facing radially away from the reference axis (1A).

2. The haptic interface (1) according to claim 1,
- wherein the haptic interface (1) further comprises a visual display screen (D), the visual display screen (D) being integrated into the top surface (T) and facing axially along the reference axis (1A).

3. The haptic interface (1) according to claim 2,
- wherein the visual display screen (D) is touch-sensitive.

4. The haptic interface (1) according to claim 2 or 3,
- wherein the haptic interface (1) is further configured for receiving (S11) configuration data, for displaying a visual information (DI) indicative of the configuration data through the visual display screen (D) and for generating the control signal for the medical imaging device (2) based on the detected manual input action and the configuration data.

5. The haptic interface (1) according to one of the claims 1 to 4,
- wherein, when the manual input action is indicative of a rotary motion (1M) about the reference axis (1A) relative to the base region (B), the haptic feedback comprises a virtual haptic rotational response.

6. The haptic interface (1) according to claim 5,
- wherein the haptic feedback comprises a virtual haptic rotational resistance response to the rotary motion (1M).

7. The haptic interface (1) according to one of the claims 1 to 6,
- wherein the haptic interface (1) comprises a circumferential light-emitting indicator (E) arranged coaxially with the reference axis (1A), positioned at a greater radial distance from the reference axis (1A) than the lateral surface (L) and facing axially along the reference axis (1A),
- wherein the haptic interface (1) is configured for providing visual feedback through the circumferential light-emitting indicator (E) in response to the detected manual input action,
- wherein the lateral surface (L) is arranged circumferential with respect to the reference axis (1A) between the circumferential light-emitting indicator (E) and the top surface (T).

8. A medical imaging device (2), comprising the haptic interface (1) according to one of the claims 1 to 7 and a gantry (20),
- wherein the base region (B) of the haptic interface (1) is integrated into a cover (V) of the gantry (20).

9. The medical imaging device (2) according to claim 8,
- wherein the gantry (20) comprises an opening (9) into which an examination object (13) is insertable along a system axis (2A) of the medical imaging device (2),
- wherein the reference axis (1A) of the haptic interface (1) is parallel to the system axis (2A).

10. The medical imaging device (2) according to claim 8 or 9, further comprising an examination table (10), the examination table (10) comprising a tabletop (12), a table base (11) and a table drive (14),
- wherein the tabletop (12) of the examination table (10) is insertable into the opening (9) along the system axis (2A) through a translational movement of the tabletop (12) relative to the table base (11) along the system axis (2A),
- wherein the table drive (14) is configured to drive the translational movement based on the control signal,
- wherein the manual input action is indicative of a rotary motion (1M) about the reference axis (1A) relative to the base region (B),
- wherein the control signal is indicative of a velocity of the translational movement, the velocity being adjustable based on a deflection angle of the rotary motion (1M).

11. The medical imaging device (2) according to one of the claims 8 to 10, further comprising a control system (21),
- wherein the control system is configured for generating the configuration data for the haptic interface (1) based on imaging workflow data.

12. A method for operating the haptic interface (1) according to one of the claims 1 to 7, the method comprising:
- detecting (S1) the manual input action of the hand based on the tactile contact between the hand and the contact region (C) of the haptic interface (1),
- generating (S2) the control signal for the medical imaging device (1) based on the detected manual input action and
- providing (S3) the haptic feedback to the hand in response to the detected manual input action.

13. The method according to claim 12, further comprising:
- receiving (S11) the configuration data,
- displaying (S12) the visual information (DI) indicative of the configuration data through the visual display screen (D),
- wherein the control signal for the medical imaging device (2) is generated based on the detected manual input action and the configuration data.

14. The method according to claim 12 or 13, further comprising:
- adjusting (S4) a velocity of a translational movement based on a deflection angle of the rotary motion (1M),
- wherein the manual input action is indicative of the rotary motion (1M) about the reference axis (1A) relative to the base region (B),
- wherein the control signal is indicative of the velocity of the translational movement.

15. The method according to claim 14,
- wherein, when the deflection angle of the rotary motion (1M) transitions across a threshold angle, a virtual rotational snap point is provided, the threshold angle corresponding to a predefined velocity threshold of the translational movement.
